# EUROPEAN PATENT SPECIFICATION

(11) **EP 2 595 625 B1**
(45) Date of publication and mention of the grant of the patent: **20.04.2016**
(21) Application number: 11729424.9
(22) Date of filing: 06.07.2011
(51) Int. Cl.: A61K 31/405, A61K 45/06, A61P 27/02

(54) **PHARMACEUTICAL COMPOSITION CONTAINING A TRYPTOPHAN DERIVATIVE**
PHARMAZEUTISCHE ZUSAMMENSETZUNG MIT EINEM TRYPTOPHANDERIVAT
COMPOSITIONS PHARMACEUTIQUES CONTENANT UN DÉRIVÉ DE TRYPTOPHANE

(30) Priority: 22.07.2010 US 366538 P; 22.07.2010 EP 10170397
(43) Date of publication of application: 29.05.2013
(73) Proprietor: Ramot at Tel-Aviv University Ltd., 6139201 Tel-Aviv (IL)
(72) Inventor: HAUPTMEIER, Bernhard, 63571 Gelnhausen (DE); KIEHM, Kevin, 60431 Frankfurt am Main (DE); PLITT, Patrick, 61352 Bad Homburg (DE); TARDT, Axel, 60599 Frankfurt am Main (DE); SZLAK-FREIER, Alda, 60431 Frankfurt am Main (DE); STREHL, Diana, 60318 Frankfurt am Main (DE)
(74) Representative: Jacobi, Markus Alexander
(86) International application number: PCT/EP2011/061356
(87) International publication number: WO 2012/010415

(56) References cited:
- WO-A1-2009/125246
- WO-A2-2009/024346
- US-A- 5 043 328
- FRYDMAN-MAROM ANAT ET AL: "Cognitive-performance recovery of Alzheimer's disease model mice by modulation of early soluble amyloidal assemblies.", 2009, ANGEWANDTE CHEMIE (INTERNATIONAL ED. IN ENGLISH) 2009 LNKD- PUBMED:19035593, VOL. 48, NR. 11, PAGE(S) 1981 - 1986, XP002601658, ISSN: 1521-3773 page 1981, column 1 page 1982 page 1986
- KANO K ET AL: "Chiral recognition of dipeptide methyl esters by an anionic beta-cyclodextrin.", August 2001 (2001-08), CHIRALITY AUG 2001 LNKD- PUBMED:11466771, VOL. 13, NR. 8, PAGE(S) 474 - 482, XP002601659, ISSN: 0899-0042 the whole document

## Description

### Field of the Invention

The present invention relates to pharmaceutical compositions containing at least one tryptophan derivative and at least one pharmaceutically acceptable cyclodextrin derivative (C). The invention also deals with a process for the preparation of such pharmaceutical compositions and to tryptophan derivatives containing compositions for different medical uses.

### Background of the invention

Since decades it is known that various amino acids play an important role for nutrition and treatment of human diseases. L-tryptophan is an essential heterocyclic amino acid which is part of various naturally occurring peptides. The amino acid typtophan forms a large variety of small peptides via its carboxylic function. Many of these peptides have physiological activities. Some small peptides (such as Trp-Pro) are known to be useful for the treatment of diseases, e. g. for amyloid-associated diseases, see US 2006/0234947. However, pharmaceutical compositions containing amino acid derivatives often are not stable during storage, in particular when formulated as aqueous solutions. Furthermore, for many pharmaceutical applications, the amount of amino acid derivative needed at the specific site of action is higher than conventional pharmaceutical formulations can provide with.

The pharmaceutical compositions of this invention comprise a tryptophan derivative which often has a molecular weight between 260 and 600 g/mol. They also contain an additional component in order to stabilize the active substance or compound, they contain a cyclodextrin derivative (C).

For the treatment of disorders and conditions of the central nervous system (CNS), various drug compounds have been developed in recent years, however many diseases, like Alzheimer's can not be treated adequately at low cost because of complex, non reproducible chemical structures, low solubilities, limited tolerability and minimal efficacy of the compounds.
metabolized in the gastric system, without providing the expected bioavailability. This is a particular issue for small peptide structures.

Furthermore, for the treatment of disorders of the eye, such as glaucoma or cataracts, no sufficiently efficient pharmaceutical compositions are available which can be easily administered. There is a high medical need to provide with pharmaceutical compositions for treatment and prevention of diseases like glaucoma, as the disease glaucoma is widely spread in all countries and can result in loss of vision, increased eye pressure and blindness. The classification of glaucoma includes the following different types of diseases:
Primary angle-closure glaucoma, secondary open-angle glaucoma, steroid-induced glaucoma, traumatic glaucoma, pigmentary dispersion syndrome, pseudo-exfoliation syndrome, secondary angel-closure glaucoma, neovascular glaucoma, uveitis and glaucoma and other not further specified eye pathologies. In addition, age-related macular degeneration is a condition which reflects features of glaucoma and leads to a progressive loss of vision, leading finally to blindness.

The definition of glaucoma includes an elevation in the intraophthalmic pressure (IOP) over a normal range. Currently available medications for the treatment of glaucoma belong to pharmacological classes including β-adrenergic blockers, cholinergic agonists, carbonic anhydrase inhibitors, alpha agonists. All medications operate under a mechanism whereby the IOP is lowered. These therapies are typically administered as eye drops. Hyper-osmotics may be administered intravenously for emergency treatment. In addition, laser therapy and surgical approaches are applied in special cases. There are different theories for the cause for the degeneration of the retinal ganglion cells including mechanical, vascular and excitotoxic mechanisms. The protein β-Amyloid (Aβ) was found to co-localize with dying retinal ganglion cells. A recent study demonstrated that inhibition of Aβ-aggregation reduces glaucomatous degeneration of retinal ganglion cells (see Li Guo et al, PNAS, 08/2007, Vol. 104, p. 13444-13449).

One important disadvantage of classical glaucoma treatment is that the ophthalmic compositions need to be applied by not very convenient routes and that they can not be stored at room temperature over a longer period of time (e.g. for weeks) without losing activity. It is known in the prior art that cyclodextrins can be used in pharmaceutical compositions.

One technical problem is that cyclodextrin containing formulations are difficult to be preserved by standard classical preservatives, since the preservatives are often coordinated by the cyclodextrin. Thus, a preservative may be inactivated by the use of cyclodextrins. Additionally, the higher the cyclodextrin concentration in the formulation, the more difficult the preserving can be. Typical concentrations of cyclodextrins in prior art pharmaceutical preparations are low, meaning that they typically vary from 0.5%-5.0%. Typical uses are mentioned e. g. in US 4,596,795, describing a tablet containing progesterone and a cyclodextrin or in WO 2009/156160 describing the CNS-compound neramexane with a cyclodextrin-derivative.

In patent US 5,043,328 some indole derivates of the following general formula are disclosed, wherein R⁷ is hydrogen or -COOH and R⁸ is hydrogen or -NH₂.

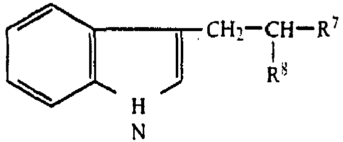

These compounds can be used in combination with fatty acids for pharmaceutical compositions, which also can contain stabilizers such as phospholipids, sugar lipids, proteins or cyclodextrins.

In WO 2009/125246, ophthalmic compositions for treatment of glaucoma and ophthalmic hypertension are described which contain an active ingredient, such as dorzolamid, which has the following structure

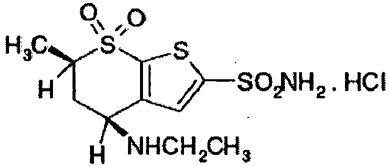

This compound can be combined with further active ingredients such a prostaglandin (see latanoprost) or timolol and can also be formulated in aqueous solutions together with a stabilizer, such as cyclodextrin.

In the document WO 2009/024346, various peptides of the structure X-Y are disclosed to be useful for the treatment of various ophthalmic diseases in which beta-amyloid plays a role. Some listings of dipeptides are provided and several diseases and types of formulations are mentioned.

In the publication of A. Frydman-Marom (Angewandte Chemie, 2009, Vol. 48, 11, 1981 - 1986), the tryptophane derivative of the following formula is described as an inhibitor of the oligomerization of beta amyloid polypeptides.

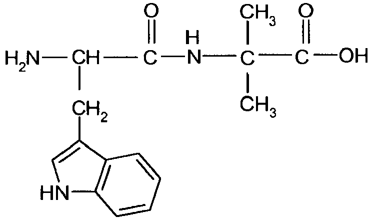

In the publication of Koji Kano (Chirality, Aug 2001, Vol. 13, 8, 474-482), several tryptophane esters are disclosed which can structurally interact with specific beta-cyclodextrins.

### Summary of the invention

It is one goal of the present invention to provide new pharmaceutical compositions which can easily be prepared, based on low-cost active ingredients, which compositions are easily applicable to humans and animals, and are well tolerated and have an acceptable long-term stability. Also the long-term stabilization of pharmaceutical, and in particular ophthalmic compositions is a goal of this invention.

The present invention in particular relates to pharmaceutical composition comprising at least one compound of formula (I) 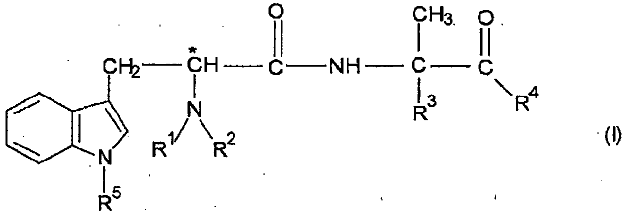 wherein the radicals denote:
R¹ is hydrogen, C₁-C₈-alkyl, C₃-C₈-cycloalkyl, C₆-C₁₄-aryl, carboxy or thiocarb;
   in particular hydrogen or C₁-C₃-alkyl,
R² is hydrogen, C₁-C₈-alkyl, C₃-C₈-cycloalkyl, C₆-C₁₄-aryl, carboxy or thiocarb;
   in particular hydrogen or C₁-C₃-alkyl,
R³ is hydrogen or C₁-C₈-alkyl,
   in particular methyl,
R⁴ is OH or O-C₁-C₈-alkyl, in particular OH,
R⁵ is hydrogen or C₁-C₈-alkyl, in particular hydrogen;
and/or a pharmaceutically acceptable salt (and/or a stereoisomeric form) thereof and at least one pharmaceutically acceptable cyclodextrin derivative (C). The composition can be e. g. a solid composition (e.g. powder, tablet), a semi-solid composition (e.g. gel) or a liquid composition (e.g. solution, suspension).

As used herein, the following definitions are applicable, unless otherwise described:
The term "C₁-C₈-alkyl" represents straight or branched chain alkyl groups, such as methyl, ethyl, propyl.
The term "O-C₁-C₈-alkyl" represents straight or branched chain -O-C₂-C₈-alkyl groups.
The term "aryl" represents an aromatic group such as e. g. phenyl or naphthyl, wherein the phenyl or naphthyl group is optionally substituted by one or two substituents, which may be the same or different, selected independently from halogen or C₁-C₆-alkyl.
The term "carboxy" represents a group -(C=O)-O-.
The term "thiocarb" represents a group -(C=S)-O-.
The term "C₃-C₈-cycloalkyl" represents a 3- to 8-membered carbocyclic ring, such as cyclopropyl, cyclohexyl and others. The term "halogen" represents fluorine, chlorine, bromine and iodine.

One further important aspect of the invention is a pharmaceutical composition comprising at least one compound of formula (I) 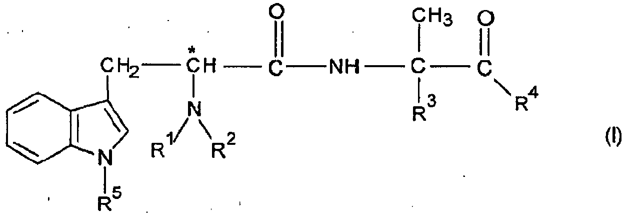 wherein the radicals denote:
R¹ is hydrogen, C₁-C₈-alkyl, C₃-C₈-cycloalkyl C₆-C₁₄-aryl, carboxy or thiocarb; in particular hydrogen or C₁-C₃-alkyl,
R² is hydrogen, C₁-C₈-alkyl, C₃-C₈-cycloalkyl, C₆-C₁₄-aryl, carboxy or thiocarb; in particular hydrogen or C₁-C₃-alkyl,
R³ is hydrogen or C₁-C₈-alkyl,
   in particular methyl,
R⁴ is OH or O-C₁-C₈-alkyl, in particular OH;
R⁵ is hydrogen or C₁-C₈-alkyl, in particular hydrogen;
   and/or a pharmaceutically acceptable salt thereof,
and at least one pharmaceutically acceptable cyclodextrin derivative (C), and optionally further components (F).

The invention also relates to a pharmaceutical composition of claim 1, wherein the composition contains at least one compound of formula (I) in which the radicals denote:
R¹ is hydrogen, C₁-C₈-alkyl, C₃-C₈-cycloalkyl or C₆-C₁₄-aryl,
R² is hydrogen, C₁-C₈-Alkyl, C₃-C₈-cycloalkyl or C₆-C₁₄-aryl,
R³ is hydrogen or C₁-C₈-alkyl,
R⁴ is OH or O-C₁-C₈-alkyl,
R⁵ is hydrogen,
   and/or a pharmaceutically acceptable salt thereof,
and comprising at least one further component (F).

A pharmaceutical composition of claim 1 is also of interest, wherein the composition contains a compound of formula (I) wherein:
R¹ is hydrogen or C₁-C₃-alkyl,
R² is hydrogen or C₁-C₃-alkyl,
R³ is hydrogen or methyl,
R⁴ is OH,
R⁵ is hydrogen,
   and/or a pharmaceutically acceptable salt thereof,
and comprises the solvent water and optionally further components (F).

The invention also relates to a pharmaceutical composition of claim 1, wherein the composition contains a compound of formula (I) wherein R¹ and R² are hydrogen, R³ is methyl and R⁴ is OH, R⁵ is hydrogen, and/or a pharmaceutically acceptable salt thereof, and comprises the solvent water and optionally further components (F).

One aspect of the invention is a pharmaceutical composition, wherein the composition contains the R-enantiomer of the compound of formula (A) 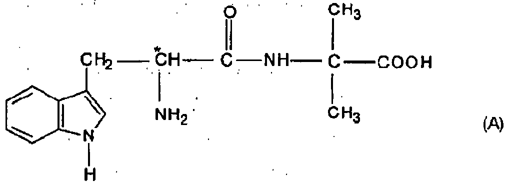 and/or a pharmaceutically acceptable salt thereof,
and at least one pharmaceutically acceptable cyclodextrin derivative (C)
and optionally a further active ingredient and/or further components (F).

Surprisingly, it was found that the solubility of compound (A) can be improved by the use of cyclodextrin derivatives (C).

The present invention also relates to a pharmaceutical composition, wherein the cyclodextrin compound (C) is selected from the group consisting of:
alpha-cyclodextrin, beta-cyclodextrin, randomly alkylated beta-cyclodextrin, 2-O-methyl-beta-cyclodextrin, heptakis-(2,6-di-0-methyl)-beta-cyclodextrin (dimethyl-beta-cyclo-dextrin), acetylated dimethyl-beta-cyclodextrin, heptakis-(2,3,6-tri-0-methyl)-beta-cyclodextrin(trimethyl-beta-cyclodextrin, 2-hydroxypropyl-beta-cyclodextrin, sulfo-alkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclo-dextrin, maltosyl-beta-cyclodextrin, beta-cyclodextrin sulphate, beta-cyclodextrin phosphate, gamma-cyclodextrin, 2-hydroxypropyl-gamma-cyclo-dextrin, sulfoalkylether-beta-cyclodextrin and sulfobutylether-beta-cyclodextrin.

The invention also relates to a pharmaceutical composition, wherein the compound of formula (I) has the R-configuration and the cyclodextrin compound (C) is selected from hydroxyalkyl-substituted beta-cyclodextrins.

The invention also relates to a pharmaceutical composition, wherein the molar ratio of the compound of formula (I) and the cyclodextrin compound (C) is in the range from 0.1:1 to 1:20, preferably from 1:1 to 1:10.

One aspect of the invention relates to a pharmaceutical composition, wherein the composition is an aqueous liquid composition comprising at least 70 % by weight of water and wherein the concentration of the compound of formula (I) is in the range from 1 mg/ml to 50 mg/ml.

In particular, the invention also relates to a pharmaceutical composition further comprising a cyclodextrin compound (C), for example in a concentration of at least 5 mg/ml, for example from 10 to 500mg/ml such as from 50 to 180 mg/ml.

The invention also relates to such a composition further comprising a hydrophilic pharmaceutical agent. The hydrophilicity can be determined by the octanol/water partition coefficient (logP), for example according to the standard methods mentioned herein. Generally, a pharmaceutical agent having an octanol/water partition coefficient of logP<1 will be considered hydrophilic. Thus, hydrophilic pharmaceutical agents may for example be selected from the group of peptides, in particular dipeptides, and in particular those of formula (I), by determining their octanol/water partition coefficient.

The invention also relates to a pharmaceutical composition of claim 1, such as an ophthalmic composition, wherein the composition is an aqueous liquid composition comprising as further component (F) a metal containing preservative. The metal containing preservative is selected from the group consisting of mercury-, silver-, copper-, tin-containing preservatives, in particular a mercury-containing preservative.

The invention also relates to a pharmaceutical composition of claim 1, such as an ophthalmic composition, wherein the composition is an aqueous liquid composition comprising as further component (F) a mercury containing preservative. The mercury containing preservative may be selected from the group consisting of thiomersal, phenylmercuric borate, 2-(ethylmercurithio)-benzoic acid, thimer fonate, phenylmercuric acetate, mercurate(1-),(2,3-dihydro-2-thioxo-5-benzoxazolecarboxylato(2-)-S)ethyl-, sodium. The invention also relates in general to an ophthalmic composition comprising such a mercury containing preservative, e. g. in a concentration from 0.1 to 0.0001 % by weight, for example from 0.01 to 0.001 % by weight.

The invention also relates to a pharmaceutical composition of claim 1, wherein the composition is an aqueous liquid composition comprising as further component (F) a metal-containing preservative, in particular the preservative thiomersal, e. g. in a concentration of 0.01 to 0.001 % by weight. The invention also relates in general to an ophthalmic composition comprising the preservative thiomersal, e. g. in a concentration of 0.01 to 0.001 % by weight.

The invention also relates to a pharmaceutical composition of claim 1 as described above for the treatment of a disorder or a disease of the central nervous system. One aspect of the invention relates to a pharmaceutical composition for the treatment of an ophthalmic disorder or disease. The invention also relates to an ophthalmic composition for neurodegenerative ophthalmic disorders or diseases.

The invention also relates to a process for preparation of a pharmaceutical composition comprising the steps of mixing together at least one compound of formula (I) 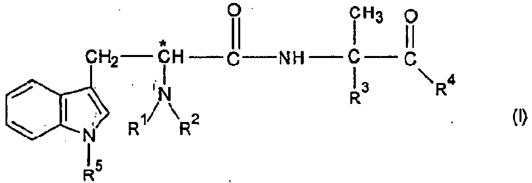 wherein the radicals denote:
R¹ is hydrogen, C₁-C₈-alkyl, C₃-C₈-cycloalkyl, C₆-C₁₄-aryl, carboxy or thiocarb;
   in particular hydrogen or C₁-C₃-alkyl,
R² is hydrogen, C₁-C₈-alkyl, C₃-C₈-cycloalkyl, C₆-C₁₄-aryl, carboxy or thiocarb;
   in particular hydrogen or C₁-C₃-alkyl,
R³ is hydrogen or C₁-C₈-alkyl,
   in particular methyl,
R⁴ is OH or O-C₁-C₈-alkyl, in particular OH,
R⁵ is hydrogen or C₁-C₈-alkyl, in particular hydrogen;
and/or a pharmaceutically acceptable salt thereof, (and/or a stereoisomeric form thereof)
and at least one pharmaceutically acceptable cyclodextrin derivative (C) and optionally further components (F).

The invention also discloses the use of a cyclodextrin compound (C) (or the compound (C) for use), in particular of a beta-cyclodextrin, for the preparation of a pharmaceutical composition for the treatment of ophthalmic diseases and to the use of a cyclodextrin compound (C), in particular of a beta-cyclodextrin, for the treatment of ophthalmic diseases. The invention also discloses the use of a hydroxyalkyl-substituted beta-cyclodextrin for the preparation of a pharmaceutical composition for the treatment of glaucoma and the use of a hydroxyalkyl-substituted beta-cyclodextrin for the treatment of glaucoma.

The invention further discloses such a use, wherein the composition is an aqueous liquid composition comprising as further component (F) a metal containing preservative. The metal containing preservative can be selected from the group consisting of mercury-, silver-, copper-, tin-containing preservatives. For example, the preservative may be silver nitrate, silver chloride, silver sulfadiazine, chitosan-copper-complexes, zinc oxide. Often the preservative is a mercury containing preservative. The mercury containing preservative may for example be selected from the group consisting of thiomersal, phenylmercuric borate, 2-(ethylmercurithio)-benzoic acid, thimer fonate, phenylmercuric acetate, Mercurate(1-), (2,3-dihydro-2-thioxo-5-benzoxazolecarboxylato(2-)-S)ethyl-, sodium.

The mercury containing preservative may be present in a concentration from 0.1 to 0.0001 % by weight, often in a concentration from 0.01 to 0.001 % by weight. For example, the preservative may be thiomersal in a concentration from 0.01 to 0.001 % by weight (of the total composition).

The invention also relates to such a use wherein the concentration of the cyclodextrin-compound (C) is at least 5 mg/ml, for example 10 to 500 mg/ml, such as 50 to 180 mg/ml.

The invention also discloses the use of a cyclodextrin compound (C) for the preparation of a pharmaceutical composition for the treatment of ophthalmic diseases in the posterior segment of the eye (including the anterior hyaloids membrane and all optical structures behind it, such as vitreous humor, retina, choroid and optic nerve).

The invention also discloses the use of a hydroxyalkyl-substituted beta-cyclodextrin for the preparation of a pharmaceutical composition for the treatment of glaucoma.

The invention also discloses the use of metal-containing preservatives for the preservation of cyclodextrin containing formulations, in particular of pharmaceutical compositions. The invention also relates to the use of metal-containing preservatives for the preservation of cyclodextrin containing formulations, in particular pharmaceutical formulations (such as ophthalmic formulations) containing a hydrophilic pharmaceutical agent. The invention also relates to the metal-containing preservative belonging to the group of silver, mercury, tin-containing preservatives.

The hydrophilic pharmaceutical agent typically exhibits an octanol/water partition coefficient of log P <1 inhibition, as outlined later.

The invention also discloses the use of metal-containing preservatives for the preservation of cyclodextrin containing formulations, in particular of pharmaceutical compositions, further comprising the use of at least one metal-containing preservative, in particular of thiomersal, wherein the pharmaceutical composition comprises a hydrophilic pharmaceutical agent, and wherein the concentration of the cyclodextrin compound (C) in the pharmaceutical composition is at least 10 mg/ml.

The compounds of the invention are usually named according to the IUPAC or CAS nomenclature system. Abbreviations which are well known to one of ordinary skill in the art may be used (e.g. "Ph" for phenyl, "Me" for methyl, "Et" for ethyl, "h" for hour or hours, and "rt" for room temperature).

The term "analog" or "derivative" is used herein in the conventional pharmaceutical sense referring to a molecule that structurally resembles a reference molecule, but has been modified in a controlled manner to replace one or more specific substituent(s) of the molecule with an alternate substituent, thereby generating a molecule which is structurally similar to the reference molecule. Synthesis and screening of analogs to identify slightly modified versions of a known compound which may have improved or biased traits (such as higher potency and/or selectivity at a specific targeted receptor type) is a drug design approach that is known in pharmaceutical chemistry.

The phrase "pharmaceutically-acceptable", as used in connection with compositions of the invention, refers to ingredients of the compositions that are physiologically tolerable and do not typically produce untoward reactions when administered to a mammal (e.g a, human). Preferably, as used herein, the term "pharmaceutically acceptable" means approved by a regulatory agency or listed in a recognized pharmacopeia for use in mammals.

The compounds of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt is not critical, provided that it is non-toxic and does not substantially interfere with the desired pharmacological activity.

The compounds of the invention having a chiral center may exist in and be isolated in optically active (such as R- or S-isomers) and racemic forms. Some compounds may exhibit polymorphism. The present invention encompasses any racemic, optically active, polymorphic, tautomeric or stereoisomeric form, or mixture thereof, of a compound of the invention, which possesses the useful properties described herein.

The compounds of formula (I) are dipeptide derivatives, which often contain at least one acidic and one basic group. As a result, the solubility in solvents like water depends on the pH-values of the composition. Often, the solubility at neutral pH is rather limited, but in acidic or alkaline compositions, the solubility is much higher. However, for many purposes, such as ophthalmic formulations, acidic or alkaline compositions are in general less desirable, e.g. due to limited tolerability.

The composition according to the invention may comprise the compound of formula (I) and/or a "pharmaceutically acceptable salt" and/or a "derivative" and/or a "polymorphic form" and/or one or several "stereoisomeric forms" of a compound of formula (I).

The composition preferably comprises at least one compound of formula (I) and/or a pharmaceutically acceptable salt thereof and also comprises at least one pharmaceutically acceptable cyclodextrin derivative (C), and optionally one or several further components (F).

The pharmaceutically acceptable salts of the compound (I) can be prepared by known methods. These salts include e. g. acid addition salts, such as salts made with hydrochloric, methylsulfonic, hydrobromic, hydroiodic, perchloric, sulfuric, nitric, phosphoric, acetic, propionic, glycolic, lactic, pyruvic, malonic, succinic, fumaric, tartaric, citric, benzoic, carbonic, cinnamic, mandelic, methanesulfonic, ethane-sulfonic, hydroxyl-ethanesulfonic, benezenesulfonic, p-toluenesulfonic, cyclohexane-sulfamic, salicyclic, p-aminosalicylic, 2-phenoxybenzoic or 2-acetoxybenzoic acid. Pharmaceutically acceptable salts also include base addition salts, e.g. using rations such as Na, K, Mg, Ca, alkylammonium or choline. All of these salts may be prepared by conventional means. The nature of the salt is not particularly critical, provided that it is non-toxic and does not substantially interfere with the desired pharmacological activity.

The invention in particular relates to aqueous liquid compositions of claim 1 containing compound (I). These are liquid preparations wherein the major liquid component is water. The compositions often contain at least 70% by weight, often at least 80 % by weight (w/w of the total composition) of water, but these aqueous liquid compositions may further comprise other liquid components, such as pharmaceutically acceptable organic co-solvents.

These co-solvents and other auxiliaries (such as preservatives, pH-regulators, sweeteners, polymers) are also named further components (F) of the composition. Examples of co-solvents are e.g. ethanol or glycerol. Such water-miscible organic co-solvents may be incorporated e.g. in order to solubilise a poorly water-soluble ingredient, such as a lipophilic substance. The term liquid composition or formulation includes liquid solutions and dispersions, such as emulsions and suspensions. Often, aqueous liquid solutions of a compound of formula (I) are preferred.

The invention also relates to semi-solid compositions. This term means a composition with low viscosity whose major liquid component is water. The semi-solid composition may comprise further components (F), such as pharmaceutically acceptable organic co-solvents, viscosity regulation polymers, pH-regulators, preservatives and emulsifiers. Examples of such liquid components are ethanol, glycerol, propylene glycol, and polyethylene glycol. Such water-miscible organic solvents (such as glycerol) may be incorporated for example in order to solubilise an insufficiently water-soluble ingredient, such as a lipophilic substance. The term semi-solid composition includes in particular gels, but also creams and ointments. In comparison to a liquid composition these formulations have an increased viscosity, compared to aqueous solutions.

The viscosity of semi-solid compositions can be controlled by using one or several polymeric components or a combination of polymers. Typical examples are acacia gum and derivatives, alginic acid and derivatives, carbomer and derivatives (e.g. carboxymethylcellulose-Na), carboxymethylcellulose and derivatives, carrageenan and derivatives, croscarmellose and derivatives, crospovidone and derivatives, dextrin and derivatives, ethylcellulose and derivatives, gelatin and derivatives, gellan gum and derivatives, guar gum and derivatives, hydroxyethyl cellulose and derivatives, hypromellose and derivatives, hydroxypropyl methylcellulose and derivatives, lecithin and derivatives, maltodextrin and derivatives, methylcellulose and derivatives, poloxamer and derivatives, polethylenglycoles and derivatives, polymethacrylates and derivatives, polyoxyethylalkylethers and derivatives, polyvinylalkohol, polyvinylpyrrolidon and derivatives (such as Kollidon, BASF), Silicon dioxide and derivatives, sodium starch glycolate and derivatives, sorbitol and derivatives, starch and derivatives and/or pre-gelatinised starch and derivatives.

The term "polymorphic form" of a compound of formula (I) means a particular crystalline or non-crystalline form of a particular compound (I) which has particular physical properties (such as particular X-ray structure) with differ from the properties of a compound having the same chemical formula.

The term "stereoisomeric form" of compound (I) is used herein in the conventional chemical sense to refer to a molecule that has the same summarizing chemical formula but differs in the structure. Typical examples are enantiomers, diastereoisomers, racemates etc.

The compositions according to the present invention contain a compound of formula (I) they comprise one or several cyclodextrin-derivatives (C). Cyclodextrin-derivatives (C) generally are oligo-saccharides composed of glucopyranose units. The unsubstituted or native cyclodextrins are usually prepared by the enzymatic degradation of starch. They are cone-like, toroidal molecules with a rigid structure and a central cavity, the size of which varies according to the cyclodextrin type.
The internal cavity of cyclodextrins is more hydrophobic and capable of accommodating both lipophilic and hydrophilic molecules in the form of inclusion complexes, thus enabling, for example, the solubilisation of poorly soluble drugs in aqueous media. Other types of complexes involving cyclodextrins have recently been identified. As used herein, a complex means an association of molecules by non-covalent interactions.

The formation of complexes takes e. g. place in solution and typically is an equilibrium process. Complexes may also occur in solid state. An inclusion complex is a structure wherein a guest molecule is either partially or completely contained within a cavity of a larger host molecule.

The three major types of pharmaceutically acceptable cyclodextrins (C) are alpha-, beta- and gamma-cyclodextrins, comprising 6, 7 and 8 glucopyranose units, respectively. In addition, a number of chemically modified cyclodextrins have been developed, often motivated by the desire to increase the water solubility and extend their usefulness as solubilising excipients. The beta-cyclodextrin-derivatives are of particular interest for the compositions according to the invention. Typical examples of such pharmaceutically acceptable cyclodextrin derivatives include the following products:
alpha-cyclodextrin (ACD), beta-cyclodextrin, randomly methylated beta-cyclodextrin, 2-O-methyl-beta-cyclodextrin (e.g. MBCD), heptakis-(2,6-di-0-methyl)-beta-cyclodextrin (dimethyl-beta-cyclodextrin), acetylated dimethyl-beta-cyclodextrin, heptakis-(2,3,6-tri-0-methyl)-beta-cyclodextrin (trimethyl-beta-cyclodextrin, sulfoalkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclodextrin, maltosyl-beta-cyclodextrin, beta-cyclodextrin sulphate, beta-cyclodextrin phosphate, gamma-cyclodextrin, sulfoalkylether-beta-cyclodextrin, and sulfobutylether-beta-cyclodextrin (SBEBCD), and hydroxyalkyl-modified cyclo-dextrins (including hydroxypropyl-beta-cyclodextrin, e.g., 2-hydroxypropyl-beta-cyclodextrin (HPBCD), or hydroxypropyl-gamma-cyclodextrin, e.g. 2-hydroxy-propylgamma-cyclodextrin (HPGCD)).

The term "cyclodextrins" includes such modified versions of the native cyclodextrins.
A typical example of a suitable grade of hydroxyalkyl-beta-cyclodextrin is amorphous, (often randomly substituted) hydroxypropyl-beta-cyclodextrin.

This often has a "Degree of Substitution" (DS) in the range of about 4.5, i.e. between approx. 4 and 5, such as the product marketed as Kleptose7i HPB (by Roquette). It is noted that the DS value, as used herein, defines the average number of substituted hydroxyl groups per anhydroglucose unit, not per cyclodextrin molecule.

Other examples of useful grades are (e.g. randomly substituted) hydroxypropyl-beta-cyclodextrin with a degree of Substitution DS in the range of about 5.6, or in the range of 2 to 4, or in the range of 5, or in the range of 6.5, respectively. An example of a suitable grade of hydroxypropyl-gamma-cyclodextrin is the product marketed as Cavasol 7i W8 HP (by Wacker Chemie, Germany).

A particularly interesting group of cyclodextrins is the group of beta-cyclodextrins, in particular the hydroypropyl-beta-cyclodextrins. An often used cyclodextrin (C) is the product Kleptose as described in the experimental part.

The amount of the compound of formula (I) in the pharmaceutical composition may be decided taking into account the desired pharmaceutical use (e.g. oral or parenteral CNS-formulations or topic ophthalmic formulations), the type of the active ingredient of formula (I) and the concentrations of the other ingredients. The concentration of the active ingredient of formula (I) may be e. g. at least 0.1 mg/ml. If the active ingredient is a compound of formula (I), the concentration in a (liquid) formulation often is in the range from 0.1 to 100 mg/ml, for example 5 to 50 mg/ml, and often from 15 to 35 mg/ml

The amount of cyclodextrin-derivative (C) in the composition may be selected taking into account the type of cyclodextrin (C) and the concentration of the active compound (I) and the pharmaceutical use. The concentration of the cyclodextrin-derivative (C) in the composition often is at least 5 mg/ml, for example 10 to 500 mg/ml, often from 50 to 180 mg/ml. If the only active ingredient is a compound of formula (I), the concentration of the cyclodextrin-derivative (C) in the composition may be in the range from about 1 to about 1000 mg/ml, for example from 10 to 500 mg/ml, often from 50 to 180 mg/ml.

Alternatively, the pharmaceutical composition may comprise a compound of formula (I) or a pharmaceutically acceptable salt thereof, in the range of 1 to 50 mg/ml.
For example, a concentration of 5-50 mg/ml (or often from 15 to 35 mg/ml) of a compound of formula (I), or a pharmaceutically acceptable salt thereof, may be formulated in such a composition. According to the present invention, for example liquid or semi solid compositions may be prepared with a molar ratio of a compound of formula (I) to cyclodextrin-derivative (C) of from 0.1 : 1 to 10 : 1.

Another embodiment of the invention may comprise a molar ratio of a compound of formula (I) to cyclodextrin-derivative (C) of from 0.3 : 1 to 3 : 1. Other embodiments may comprise a molar ratio of about 0.6 : 1 to 2 : 1.

It was found that such molar ratios lead to a remarkably high degree of taste masking, which may be associated with the spontaneous formation of a soluble complex between the cyclodextrin molecule and the drug of formula (I).

Moreover, the potentially unpleasant smell of the active compound of formula (I) can substantially be reduced. The degree of taste-masking is surprising in view of the high water-solubility of the compounds of formula (I). It is surprising that apparently a complexation achieves effective taste/smell-masking for a peptide compound without eliminating other properties of the drug substance.

After oral or topic (e.g. into the eye) administration, the compound of formula (I) is rapidly absorbed from the composition and becomes bioavailable.

The addition of one or more further components (F) which improve the character of the pharmaceutical composition is also possible. For example, for oral compositions, one or more sweeteners may be incorporated into the composition. Furthermore, one or more excipients selected from the group of flavours, flavour enhancers, and taste masking agents may be added. Typical sweeteners are natural or synthetic compounds which have a sweet taste and are physiologically acceptable. Examples of natural sweeteners include common sugars and sugar alcohols such as sucrose, glucose, fructose, maltose, maltitol, xylitol, lactitol, mannitol, and sorbitol. A sugar alcohol may be used to improve the flavour of the composition of the invention, for example sorbitol. A useful concentration range for sorbitol or other sugars and sugar alcohols is from about 5 % (w/v) to about 25 % (w/v), a 10 % (w/v) may also be used. Useful artificial sweeteners include saccharin-sodium, Saccharin, sodium cyclamate, acesulfame K, neohesperidine dihydrochalcone, and aspartame, as well as any other sweeteners whose safety in human use is established.
Appropriate concentrations depend on the individual sweetener which is selected, but also on the specific cyclodextrin which is chosen. For example, hydroxypropyl-beta-cyclodextrin already provides for a rather sweet taste so that the addition of a sweetening agent may not increase the palatability of the formulation any further.

Suitable flavors which may further improve the taste of cyclodextrin-containing aqueous compositions of compounds of formula (I) (including compound (A) and pharmaceutically acceptable salts thereof) include grape, orange, peppermint, spearmint, cherry, liquorice, and aniseed. In particular, peppermint flavours are physicochemically and organoleptically well-compatible with the key components of the composition of the invention and may lead to palatable formulations.

For liquid compositions, the preservation of the active compound of formula (I) is very important. It is possible to formulate the composition without any additional preservative. In one embodiment, the composition of the invention is substantially free of preservatives. In this context, the term "substantially" means that preservatives are not detectable in the composition, or only in concentrations which are generally considered irrelevant with regard to any preservation effects. The pharmaceutical composition may optionally comprise as further component (F) at least one preservative. Whether a composition is effectively preserved may be determined according to tests known in the art.

The pharmaceutical composition can also contain as further component (F) a preservative such as benzalkonium chloride, cetylpyridium chloride, cetrimide, cetyl trimethylammonium bromide, benzethonium chloride, chlorhexidine gluconate, ethanol, isopropanol, propylen glycol, butylparaben, ethylparaben, methylparaben, propylparaben, sorbic acid, benzoic acid, thiomersal, organomercury components, chlorobutanol and/or benzyl alcohol. As further component (F) and preservative the compound thiomersal and combinations of thiomersal with a second preservative are of particular use for the pharmaceutical compositions.

It was surprisingly found, that certain further components (F) are particularly useful for the preservation of ophthalmic formulations (such as solutions of gels), which comprise a compound of general formula (I). It can be particularly difficult to preserve formulations against microorganisms (e.g. bacteria and fungi), if the formulation contains a cyclodextrin derivative.

From different tests it can be seen that a sufficient microbial preservation of ophthalmic formulations is not possible with benzalkonium chloride, cetrimide and chlorhexidine according to the European standards (Pharmacopeia 7.0) and US standards (US-Pharmacopeia).

It can be shown that in particular the aromatic Hg-containing compounds, such as thiomersal exhibit a sufficient activity against Candida albicans, Aspergillus niger, Pseudomonas aeruginosa and Staphylococcus aureus, which meet the compendia requirements.

Even in a low concentration of 0.001 to 0.01 % by weight of thiomersal in the aqueous formulation, a preservation against the organisms Candida albicans, Aspergillus niger, Pseudomonas aeruginosa and Staphylococcus aureus for 28 days (and more) can be achieved.

Also the combination of thiomersal with a second preservative, such as benzalkonium chloride, was found to be applicable for the preservation of ophthalmic formulations, and in particular those formulations comprising a tryptophane derivative of formula (I), in particular compound (A), and a cyclodextrin-component (in particular Kleptose).

The invention also relates to the use of thiomersal for the preservation of a pharmaceutical composition, in particular an ophthalmic formulation, e.g. as those described above.

The pharmaceutical composition can as further component (F) also contain a pH-regulator, e. g. to improve the stability. Typical examples are selected from the group consisting of physiologically acceptable acids, bases, and acidic and alkaline salts. For example, the combination of citric acid and sodium citrate may be used for buffering the pH of the composition at a value selected in the range from about pH 4 to about pH 8. In one embodiment, the pH of the pharmaceutical composition may be adjusted to a value from pH 2 to pH 12 (e. g. for injection solutions) or to a value from pH 3 to pH 9 (e. g. for oral or ophthalmic solutions), using pharmaceutically acceptable buffering agents or mixtures (such as citric acid/sodium citrate buffer, phosphate buffer, tromethanol buffer, boric acid buffer, amino acid buffer or acetate buffer).

A very useful pH of the pharmaceutical composition for ophthalmic applications is a value from pH 5 to pH 6.5, often from 5 to 6.

Further components (F) which are routinely used in pharmaceutical formulations may be incorporated as appropriate to adjust the composition to the specific requirements of a particular drug compound, or to a specific use or target population. Typical examples of further components (F) are thickeners such as soluble gums, including carrageenan, alginate, xanthan, and soluble cellulose esters; colouring agents; stabilizers, such as antioxidants, or crystallization inhibitors, such as glycerol, propylene glycol, or polyvinylpyrrolidone.

The compounds of formula (I) can be used for the prevention and/or treatment of neurodegenerative conditions or diseases, among these ophthalmic conditions or diseases associated with neurodegeneration.

They can be used e. g. for the following diseases:
Alzheimer's disease, Creutzfeld-Jakob's syndrome/disease, bovine spongiform encephalopathy (BSE), diseases involving β-amyloid and/or tauopathy, motor neuron diseases, amyotrophic lateral sclerosis (ALS), olivoponto-cerebellar atrophy, post-operative cognitive deficit (POCD), systemic lupus erythematosus, systemic clerosis, Sjogren's syndrome, Neuronal Ceroid Lipofuscinosis, neurodegenerative cerebellar ataxias, Parkinson's disease, Parkinson's dementia, cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, convulsions, epileptic convulsions, epilepsy, temporal lobe epilepsy, myoclonic epilepsy, inner ear insult, tinnitus, L-dopa-induced dykinesias, dyskinesias, chorea, Huntington's chorea, athetosis, dystonia, stereotypy, ballism, tardive dyskinesias, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, major depressive disorder, depression, bipolar manic-depressive disorder, irritable bowel syndrome (IBS), migraine, multiple sclerosis (MS), muscle spasms, pain, chronic pain, acute pain, inflammatory pain, schizophrenia, spasticity, Tourette's syndrome, sleep disorders, anxiety disorder, obsessive-compulsive disorder, panic disorder, posttraumatic stress disorder, social phobia, phobic disorders and schizophreniform disorder.

Also age related macular degeneration can be treated by the compounds of formula (I).

Optionally, the composition may further comprise another active ingredient which is not a compound of formula (I). The invention also relates to a combination to be co-administered to the living human or animal a therapeutically effective amount of a compound (I) as described above in combination with at least one additional pharmaceutical agent which is effective in treating e. g. the ophthalmic condition, wherein the combination of the compound (I) and the at least one additional pharmaceutical agent is effective in treating the condition.

The additional pharmaceutical agent is e.g. selected from medications administered to treat eye diseases containing anti-glaucoma drugs, antibiotics, anti-inflammatory drugs, steroids, anti-allergic drugs and artificial tear fluid. As anti-glaucoma drugs, IOP-lowering drugs are of particular interest.

Such an additional pharmaceutical agent is e. g. selected from:
acetazolamide, diclofenamide, carteolol, timolol, metipranolol, betaxolol, pindolol, levobunolol, brimonidine, clonidine, pilocarpine, carbachol, dipivefrine, apraclonidine, brinzolamide, dorzolaminde, bimatroprost, travaprost, latanoprost, chlortetracycline, ciprofloxacine, ofloxacine, fusidinic acid, gentamicine, kanamycine, levofloxacine, lomefloxacine, oxytetracycline, natamycine, azidamfenicole, chloramphenicole, tobramycine, erythromycin, polymyxin-B, acaclovir, trifluridine, betamethasone, dexamethasone, fluorometholone, hydrocortisone, prednisolone, rimexolone, cromoglicate, azelastine, lodoxamide, emedastine, nedocromile, levocabstine, olopatadinea, ketoifene, hypromellose, carbomere, hyaluronate, carmellose, hypromellose, povidone, hyetellose, polivinylalcohole, dexpanthenole, tetryzoline, troxerutine, tramazoline, naphazoline, xylometazoline, phenylephrine and antazoline.

The compound of formula (I) or the combination product is e.g. administered once a day, twice a day or three times a day. Often it is administered chronically. In one embodiment, the composition is administered in the form of eye drops, eye creams, and intraophthalmic depot formulations. The composition can also be administered in an immediate or modified release formulation. The compound of formula (I) and the additional pharmaceutical agent can be administered separately or conjointly.

Often, the pharmaceutical compositions disclosed herein may be administered in addition to one or more pressure lowering (IOP-lowering) drugs, for example IOP lowering drugs as used in conventional glaucoma treatment.

It has been surprisingly found that the combination of a compound of formula (I) and/or its pharmaceutically acceptable salts and at least one cyclodextrin derivative (C) is particularly useful in aqueous media.

A complex formation may take place with several types of native cyclodextrins or synthetic cyclodextrins, including beta- and gamma-cyclodextrin, in particular hydroxyalkyl-beta- or hydroxypropyl-beta-cyclodextrin. The complex formation takes place spontaneously in aqueous solutions at normal room temperature, which is in contrast to many other known cyclodextrin complexes whose preparation requires the application of heat and/or long stirring times.

The preparation of the composition according to the invention is technically easy, quick and cost-efficient. For the preparation of the compositions, the components are e. g. weighed and the compound of formula (I) is combined (mixed) with measured amounts of the cyclodextrin-derivative (C) and water and optionally further components (F), optionally followed by stirring until dissolution occurs. The mixture may be agitated and/or heated. The solution may be further processed by filtration or centrifugation to remove residual particles. If a solid formulation is desired, the solution may be dried, such as by spray drying or freeze drying.

The following experiments were carried out in order to further illustrate the invention.

### Example 1

Five pharmaceutical compositions were prepared using the compound (A), which has the chemical name 2-[2-Amino-3-(1H-indol-3-yl)-propionylamino]-2-methyl-propionic acid

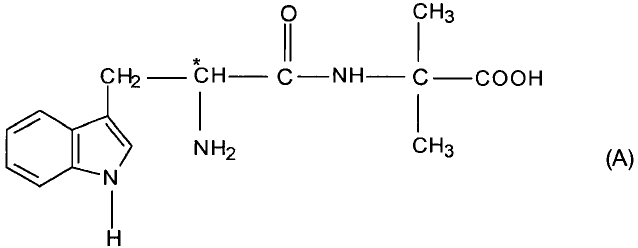

This compound (A) and its two enantiomers (R and S) were all prepared by classical chemical synthesis. The compound, and in particular its R-enantiomer, was tested in various compositions for CNS-applications and for ophthalmic uses.

In the following tests, the R-enantiomer of compound (A) was used.
The compound (A) showed an aqueous solubility, at pH 7.2, of about 14 mg/ml.
The compound (A) showed an aqueous solubility, at pH 7, of about 10 mg/ml.
The compound (A) showed an aqueous solubility, at pH 6, of about 10 mg/ml.
The compound (A) showed an aqueous solubility, at pH 4.6, of about 14 mg/ml.

At acidic conditions, the solubility of the compound (A) is higher.
At alkaline pH-values, the solubility of the compound (A) is also better.

The solubility of the R-enantiomer of compound (A) was determined as a function of the pH-value by using UV-spectroscopy to quantify the concentration of the compound in a series of aqueous media. It was found that from pH 4.5 to 7 the solubility is particularly low.

The octanol/water partition coefficient logP of the compounds of the invention can be determined by accepted standard methods known to the person skilled in the art, such as OECD (1995),Test No. 107: Partition Coefficient (n-octanol/water): Shake Flask Method, OECD Guidelines for the Testing of Chemicals, Section 1: Physical-Chemical properties, OECD Publishing. doi: 10.1787/9789264069626-en.
The logP value may be determined according to the draft OECD guideline OECD (2000), OECD Draft guideline for the Testing of Chemicals: 122 Partition Coefficient (n-Octanol/Water): pH-Metric Method for Ionisable Substances. For Example, logP of compound (A) was investigated using OECD (2000) OECD Draft guideline for the Testing of Chemicals: 122. The sample was titrated in two triple titrations from pH 1.9 to pH 10.9 at concentrations of 1.3 to 1.9mM in various ratios of octanol / water. The apparent sample pKas in octanol / water were found to have no measurable shift from their aqueous values, hence the sample logP is lower than -1.

### Example 2

The following compositions I to V were prepared as solutions with a pH-value of about 7:

| | Composition I [mg/g] | Composition II [mg/g] |
|---|---|---|
| Compound (A) | 8.0 | 8.0 |
| Glycerol | 28 | 28 |
| Carbopol 974 P | 1.3 | 1.6 |
| Trometamol | 1.0 | 1.2 |
| Water | ad 1000 | ad 1000 |

| | Composition III [mg/ml] | Composition IV [mg/ml] |
|---|---|---|
| Compound (A) | 30 | 30 |
| Kleptose | 160 | 160 |
| Disodium hydrogen phosphate | 4.15 | 4.15 |
| Sodium dihydrogen phosphate | 0.29 | 0.29 |
| Sodium chloride | 2.0 | 2.0 |
| Methocel E4M | - | 5.0 |
| Water | Qsp | qsp |

### Composition V [mg/g]

| | |
|---|---|
| Compound (A) | 30 |
| Kleptose | 160 |
| Glycerol (99%) | 5.0 |
| Gelrite | 2.0 |
| 1M NaOH | 0.015 mL ; water qsp |
| Water | ad 1000 |

As it easily can be seen, compositions I and II (gel) contain 8.0 mg of compound (A) per ml of the composition. Composition I and II do not contain a cyclodextrin derivative (C).

The compositions III, IV and V contain 30 mg of compound (A) per ml of the composition. All three compositions III (solution), IV (gel) and V (in situ gel) also contain a considerable amount of a cyclodextrin derivative (C). Various ratios of compound of formula (I) to cyclodextrine derivative (C) were tested.

### Example 3

In the following table the major components of the pharmaceutical compositions tested are described in more detail:

| | |
|---|---|
| Excipient | Manufacturer/Supplier |
| Kleptose HPB | Roquette; Hydroxypropyl-beta-cyclodextrin |
| Glycerol 99% | Merck KGaA |
| Gelzan CM | Sigma Inc. (USA), Gellan Gum |
| Carbomer 974 P | Noveon, Carboxymethyl cellulose-Na |
| Methocel E4M | Colorcon, Methylhydroxy-propyl-cellulose |
| Benzalkonium chloride solution 50% | Euro OTC |
| Kollidon 90 F | BASF, Polyvinylpyrrolidone |
| Trometamol | Merck KGaA, Tris(hydroxymethyl)-aminomethan |

The water used was water for injection.
The following properties were found for the pharmaceutical compositions I to V:

| Composition | Compound A [mg/mL] | pH | Osmolality [mosmol/kg] | Viscosity [mPa*s] |
|---|---|---|---|---|
| Composition I | 8.0 | 6.4 | 315 | 40.8 |
| Composition II | 8.0 | 6.4 | 310 | 192.3 |
| Composition III | 30.0 | 6.9 | 305 | 1.5 |
| Composition IV | 30.0 | 6.9 | 320 | 18.1 |
| Composition V | 30.0 | 7.1 | 215 | 71.3 |

The five pharmaceutical compositions I to V, all containing the active ingredient (A), were applied to the eyes of test animals (dark agouti rats (pigmented) and to albino rats).
At different points of time after the administration of the compositions into the eye a microdissection of the eye of the animal was performed. Tissue samples were collected and a chemical dissolution and analysis was made for different compartments of the eye of the test animals.

With all five pharmaceutical compositions I to V, a significant amount of compound of formula (A) was delivered to the retina of the test animals. The evaluation of the results obtained from the Retina, the Vitreous, the Choroid and the Plasma of the test animals showed that the amounts of the compound (A) in the various compartments of the eye were significantly higher, when the compositions III, IV and V were used than when the pharmaceutical compositions I and II were applied.

For example, the amounts of compound (A) in the Retina of dark agouti rats was more than three times higher when composition III was applied (or when composition V was applied) than by applying compositions I or II to the eye of the test animal.

Therefore, a much better treatment of glaucoma can be achieved with compositions III to V than with compositions I and II.

### Example 4

For further pharmacological testing, three additional pharmaceutical compositions VI, VII and VIII were prepared by using the following components:

| | Composition VI [mg/ml] | Composition VII [mg/ml] |
|---|---|---|
| Compound (A) | 20 | 20 |
| Kleptose | 160 | 160 |
| Citric acid anhydrous | 1.30 | 1.30 |
| Sodium citrate dehydrate | 7.70 | 7.70 |
| Methocel E4M | - | 5.0 |
| Water | qsp | qsp |

| | Composition VIII [mg/ml] |
|---|---|
| Compound (A) | 20 |
| Kleptose | 160 |
| Glycerol | 5.0 |
| Gelrite | 2.0 |
| 1 M NaOH | - |
| Water | qsp |

The pharmaceutical compositions VI (solution), VII (gel) and VIII (in situ gel) can be characterized by the following characteristics:

| Formulation | Compound A [mg/mL] | pH | Osmolality [mosmol/kg] |
|---|---|---|---|
| Composition VI | 20.0 | 5.7 | 288 |
| Composition VII | 20.0 | 5.7 | 266 |
| Composition VIII | - 20.0 | 5.7 | 245 |

All three compositions VI, VII and VIII were tested in test animals (rats as described above). For all three compositions, significant amounts of compound (A) were delivered to the retina of the animals. The ratio of compound of formula (I) to cyclodextrine derivative (C) was 1:8. Even by using smaller concentrations of the active ingredient (A) in compositions VI, VII and VIII, and by using the preferred pH-value range of 5.6 to 6.2, very good test data were obtained.

### Example 5

For application to the human eye, the following ophthalmic composition IX was prepared:

| | Composition IX [mg/ml] | |
|---|---|---|
| Compound (A) | 20 | |
| Kleptose | 110 | |
| Citric acid anhydrous | 0.80 | |
| Sodium citrate dehydrate | 10.6 | |
| Methocel E4M | 5.0 | |
| Sodium chloride | 1.5 | |
| Water | Qsp | |

This composition had a viscosity of 32 mPas (using a cone-plate rheometer, C60/1°).

The release rate of compound (A) from this composition was tested in a Franz cell study (Supor-200 membrane). After 60 minutes, already 60 % of the active compound were released.

An additional Composition IXa was prepared and tested comprising:

| | Composition IXa [mg/ml] | |
|---|---|---|
| Compound (A) | 20 | |
| Kleptose | 110 | |
| Citric acid anhydrous | 0.80 | |
| Sodium citrate dehydrate | 10.6 | |
| Methocel E4M | 5.0 | |
| Glycerol | 1.5 | |
| Water | Qsp | |

### Example 6

In addition, the stability of the aqueous compositions VI to VIII at various temperatures (5°C, 25°C and 40°C) was investigated. Even after four weeks of storage, the compositions VI to VIII containing compound (A) only showed moderate degradation.

### Example 7

For further stability testing, the following compositions were prepared:
a) 10 mg compound (A) per ml, with 0.1 mg sodium benzoate per ml;
b) 10 mg compound (A) per ml, with 0.2 mg sodium benzoate per ml;
c) 100 mg compound (A) per ml, with 0.1 mg sodium benzoate per ml;
d) 100 mg compound (A) per ml, with 0.2 mg sodium benzoate per ml;
e) 10 mg compound (A) per ml, with 0.1 mg sodium benzoate per ml and 10 % (v/v) of propylene glycol;
f) 10 mg compound (A) per ml, with 0.2 mg sodium benzoate per ml and 10 % (v/v) of propylene glycol.

For all compositions, the compound (A) was dissolved in citric acid solution and completed to the required volume with citric buffer to a pH-value of 3.

Then, the preservatives were added. The six solutions were first tested for one week at room temperature.

None of the formulations showed any change after one week at room temperature, the optical appearance, the pH-value and the odor remained unchanged. Then they were stored for several weeks at temperatures of 25° C with 60 % humidity and at 5° C. The compositions with 10 mg compound (A) were stable for several months.

### Example 8

In the further series of tests, the compatibility of the compound (A) with other typical ophthalmic excipients was investigated. It was found that compound (A) in aqueous solutions at pH 5.5 showed no incompatibilities with many further components (F), such as hypromellose, carbomer, Gellan gum and various preservatives.

### Example 9

In a male Dark Aguti rat model, glaucoma is produced by injection of hypertonic saline into episcleral veins of one eye to induce increased ophthalmic pressure (chronic ophthalmic hypertension), while the opposite eye serves as a control. In treatment groups, various doses of compound (A) of the instant invention are injected intravitreally (in 50 µl volume) at the time of glaucoma induction and in some groups the administration continues for following 7 days to see whether such extended treatment results in increased efficacy.

The extent of retinal ganglion cell (RGC) apoptosis at 3 weeks and 6 weeks after chronic ophthalmic hypertension (OHT) induction is assessed in each animal by dynamic confocal scanning laser ophthalmoscopy and fluorescent-labeled Annexin V.

Animals are sacrificed after 3 and 6 weeks and their eyes are enucleated and fixed in 4% para-formaldehyde overnight. Afterwards, retinas are separated for assessing apoptosis related changes, for example visualized with FITC Annexin V kit (BD Biosciences, Franklin Laker (USA).

In animals treated with low doses of compound (A), there is a strong decrease in RGC apoptosis compared to animals treated with the vehicle only. This shows that compound (A) is useful for the treatment of glaucoma.

The equlilibrium solubility of compound (A) was tested in a citrate buffer saline at pH6 in presence of different cyclodextrins by the shake flask method.
The beta-cyclodextrins HPBCD and MBCD showed the best solubility improvement of the tested cyclodextrin derivatives.

| | Solubility of compound (A) [mg/mL] |
|---|---|
| 110 mg/ml HPBCD (Kleptose HPB, Roquette) | 27.9 |
| 110 mg/ml HPGCD (Cavasol W8 HP Pharma, ISP) | 16.2 |
| 110 mg/ml MBCD (Kleptose Crysmeb, Roquette) | 28.7 |
| 100 mg/ml ACD (α-Cyclodextrin, Fluka) | 12.7 |

All these compositions can advantageously be used for the treatment of the various ophthalmic diseases described above.

### Example 10

In male Dark Aguti rats, an aqueous formulation comprising 20 mg/mL of Compound (A) and 160 mg/mL Kleptose was applied (one time) to one eye, while the opposite eye served as a control.

In other male Dark Aguti rats, an aqueous formulation comprising 8 mg/mL of Compound (A) but containing no Kleptose (cyclodextrin) was applied (one time) to one eye, while the opposite eye served as a control. In the treatment groups, the formulations comprising compound (A) were injected intra-vitreally (in 10 µl volume instillation) and the concentrations of compound (A) were measured in the retina of the animals (after 5 minutes). The mean concentrations of compound (A) are as follows:

| | | |
|---|---|---|
| Formulation with Compound A | 0,005 % | of dose applied, |
| Formulation with Compound A + Cyclodextrin | 0,2 % | of dose applied. |

This shows the surprising effect of the combined use of compound (A) with a cyclodextrin, that a 40-fold better permeation can be achieved, even by applying only a 2.5-fold dosis of the drug compound.

### Example 11

For testing of the long term preservation of the ophthalmic formulations with metal-containing preservatives, the pharmaceutical compositions VI of example 4 was modified by adding thiomersal in a concentration of 0.01 % by weight in the aqueous formulation.

A long-time preservation against the organisms Candida albicans, Aspergillus niger, Pseudomonas aeruginosa and Staphylococcus aureus after 28 days was observed with a reduction of all for organisms of at least log 5.

By using the preservative sorbic acid (0.3 % by weight) instead of thiomersal in the above mentioned ophthalmic formulation, already after 14 days an insufficient preservation against Candida albicans can be observed.

### Example 12

For testing the influence of the cyclodextrin content in ophthalmic formulations, the solubility of Compound (A) was tested. The equilibrium solubility (at 20°C) of compound (A) in water without cyclodextrin was measured as 11.6 mg/ml.

The equilibrium solubility (at 20°C) of compound (A) in water containing 65 mg/ml cyclodextrin (HPBCD) was measured as 20.2 mg/ml. The equilibrium solubility (at 20°C) of compound (A) in water containing 165 mg/ml cyclodextrin was measured as 41.2 mg/ml.

## Claims

1. Pharmaceutical composition comprising at least one compound of formula (I) 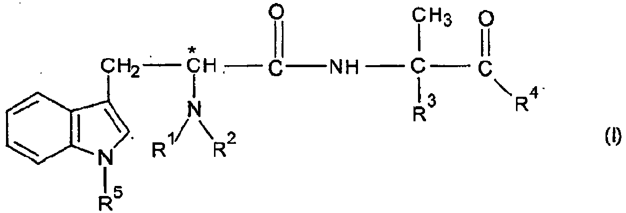 wherein the radicals denote:
R¹ is hydrogen, C₁-C₈-alkyl, C₃-C₈-cycloalkyl, C₆-C₁₄-aryl, carboxy or thiocarb; in particular hydrogen or C₁-C₃-alkyl,
R² is hydrogen, C₁-C₈-alkyl, C₃-C₈-cycloalkyl, C₆-C₁₄-aryl, carboxy or thiocarb; in particular hydrogen or C₁-C₃-alkyl,
R³ is hydrogen or C₁-C₈-alkyl, in particular methyl,
R⁴ is OH or O-C₁-C₈-alkyl, in particular OH;
R⁵ is hydrogen or C₁-C₈-alkyl, in particular hydrogen;
and/or a pharmaceutically acceptable salt thereof, and/or a stereoisomeric form thereof, and at least one pharmaceutically acceptable cyclodextrin derivative (C) and optionally further components (F).

2. Pharmaceutical composition according to claim 1, wherein the composition contains at least one compound of formula (I) in which the radicals denote:
R¹ is hydrogen, C₁-C₈-alkyl, C₃-C₈-cycloalkyl or C₆-C₁₄-aryl,
R² is hydrogen, C₁-C₈-alkyl, C₃-C₈-cycloalkyl or C₆-C₁₄-aryl,
R³ is hydrogen or C₁-C₈-alkyl,
R⁴ is OH or O-C₁-C₈-alkyl,
R⁵ is hydrogen or C₁-C₈-alkyl, in particular hydrogen,
and/or a pharmaceutically acceptable salt thereof,
and comprises at least one further component (F).

3. Pharmaceutical composition according to claim 1 or 2, wherein the composition contains a compound of formula (I) wherein
R¹ is hydrogen or C₁-C₃-alkyl,
R² is hydrogen or C₁-C₃-alkyl,
R³ is hydrogen or methyl,
R⁴ is OH,
R⁵ is hydrogen or C₁-C₈-alkyl, in particular hydrogen,
and/or a pharmaceutically acceptable salt thereof,
and comprises the solvent water and optionally further components (F).

4. Pharmaceutical composition according to any of claims 1 to 3, wherein the composition contains a compound of formula (I) wherein R¹ and R² are hydrogen,
R³ is methyl, R⁴ is OH and R⁵ is hydrogen, and/or a pharmaceutically acceptable salt thereof, and comprises the solvent water and optionally further components (F).

5. Pharmaceutical composition according to any of claim 1 to 4, wherein the composition contains the R-enantiomer of the compound of formula (A) 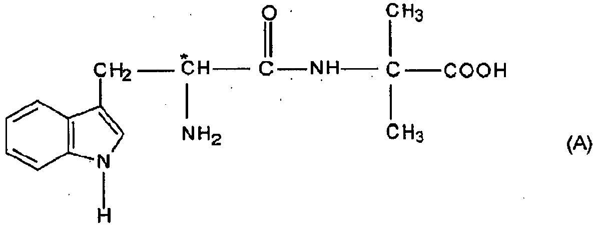 and/or a pharmaceutically acceptable salt thereof,
and at least one pharmaceutically acceptable cyclodextrin derivative (C) and optionally a further active ingredient and/or further components (F).

6. Pharmaceutical composition according to any of claim 1 to 5, wherein the cyclodextrin compound (C) is selected from the group consisting of:
alpha-cyclodextrin, beta-cyclodextrin, randomly alkylated beta-cyclodextrin, 2-0-methyl-beta-cyclodextrin, heptakis-(2,6-di-0-methyl)-beta-cyclodextrin (dimethyl-beta-cyclo-dextrin), acetylated dimethyl-beta-cyclodextrin, heptakis-(2,3,6-tri-0-methyl)-beta-cyclodextrin(trimethyl-beta-cyclodextrin, 2-hydroxypropyl-beta-cyclodextrin, sulfo-alkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclo-dextrin, maltosyl-beta-cyclodextrin, beta-cyclodextrin sulphate, beta-cyclodextrin phosphate, gamma-cyclodextrin, 2-hydroxypropyl-gamma-cyclodextrin, sulfoalkylether-beta-cyclodextrin and sulfobutylether-beta-cyclodextrin.

7. Pharmaceutical composition according to any of Claims 1 to 6, wherein the compound of formula (I) has the R-configuration and the cyclodextrin compound (C) is selected from hydroxyalkyl-substituted beta-cyclodextrins.

8. Pharmaceutical composition according to any of claims 1 to 7, wherein the molar ratio of the compound of formula (I) and the cyclodextrin compound (C) is in the range from 0.1:1 to 1:20.

9. Pharmaceutical composition according to any of claims 1 to 8, wherein the composition is an aqueous liquid composition comprising at least 70 % by weight of water and wherein the concentration of the compound of formula (I) is in the range from 1 mg/ml to 50 mg/ml.

10. Pharmaceutical composition according to any of claims 1 to 9, wherein the composition is an aqueous liquid composition comprising as further component (F) a metal-containing preservative, in particular the preservative thiomersal.

11. Pharmaceutical composition according to any of claims 1 to 10 for the treatment of a disorder or a disease of the central nervous system.

12. Pharmaceutical composition according to any of claims 1 to 10 for the treatment of an ophthalmic disorder or disease.

13. Process for preparation of a pharmaceutical composition comprising the steps of mixing together at least one compound of formula (I) 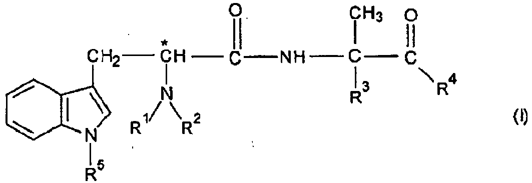 wherein the radicals denote:
R¹ is hydrogen, C₁-C₈-alkyl, C₃-C₈-cycloalkyl, C₆-C₁₄-aryl, carboxy or thiocarb; in particular hydrogen or C₁-C₃-alkyl,
R² is hydrogen, C₁-C₈-alkyl, C₃-C₈-cycloalkyl, C₆-C₁₄-aryl, carboxy or thiocarb; in particular hydrogen or C₁-C₃-alkyl,
R³ is hydrogen or C₁-C₈-alkyl, in particular methyl,
R⁴ is OH or O-C₁-C₈-alkyl, in particular OH;
R⁵ is hydrogen or C₁-C₈-alkyl, in particular hydrogen;
and/or a pharmaceutically acceptable salt thereof, and/or a stereoisomeric form thereof,
and at least one pharmaceutically acceptable cyclodextrin derivative (C) and optionally further components (F).

## Patentansprüche

1. Pharmazeutische Zusammensetzung umfassend mindestens eine Verbindung der Formel (I) 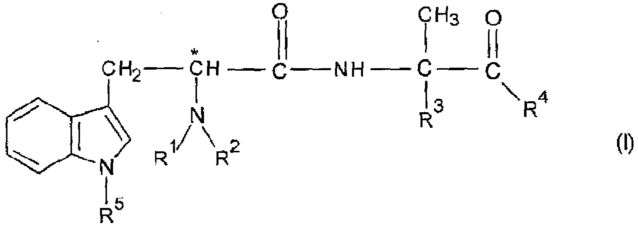 wobei die Reste bedeuten:
R¹ ist Wasserstoff, C₁-C₈-Alkyl, C₃-C₈-Cycloalkyl, C₆-C₁₄-Aryl, Carboxy oder Thiocarb; insbesondere Wasserstoff oder C₁-C₃-Alkyl,
R² ist Wasserstoff, C₁-C₈-Alkyl, C₃-C₈-Cycloalkyl, C₆-C₁₄-Aryl, Carboxy oder Thiocarb; insbesondere Wasserstoff oder C₁-C₃-Alkyl,
R³ ist Wasserstoff oder C₁-C₈-Alkyl, insbesondere Methyl,
R⁴ ist OH oder O-C₁-C₈-Alkyl, insbesondere OH;
R⁵ ist Wasserstoff oder C₁-C₈-Alkyl, insbesondere Wasserstoff;
und/oder ein pharmazeutisch verträgliches Salz davon und/oder eine stereoisomere Form davon und mindestens ein pharmazeutisch verträgliches Cyclodextrin-Derivat (C) und optional weitere Komponenten (F).

2. Pharmazeutische Zusammensetzung gemäß Anspruch 1, wobei die Zusammensetzung mindestens eine Verbindung der Formel (I) enthält, bei der die Reste bedeuten:
R¹ ist Wasserstoff, C₁-C₈-Alkyl, C₃-C₈-Cycloalkyl oder C₆-C₁₄-Aryl,
R² ist Wasserstoff, C₁-C₈-Alkyl, C₃-C₈-Cycloalkyl oder C₆-C₁₄-Aryl,
R³ ist Wasserstoff oder C₁-C₈-Alkyl,
R⁴ ist OH oder O-C₁-C₈-Alkyl,
R⁵ ist Wasserstoff oder C₁-C₈-Alkyl, insbesondere Wasserstoff,
und/oder ein pharmazeutisch verträgliches Salz davon,
und umfasst mindestens eine weitere Komponente (F).

3. Pharmazeutische Zusammensetzung gemäß Anspruch 1 oder 2, wobei die Zusammensetzung eine Verbindung der Formel (I) enthält, wobei
R¹ Wasserstoffoder C₁-C₃-Alkyl ist,
R² Wasserstoff oder C₁-C₃-Alkyl ist,
R³ Wasserstoff oder Methyl ist,
R⁴ OH ist,
R⁵ Wasserstoff oder C₁-C₈-Alkyl, insbesondere Wasserstoff, ist,
und/oder ein pharmazeutisch verträgliches Salz davon,
und umfasst das Lösungsmittel Wasser und optional weitere Komponenten (F).

4. Pharmazeutische Zusammensetzung gemäß einem beliebigen der Ansprüche 1 bis 3, wobei die Zusammensetzung eine Verbindung der Formel (I) enthält, wobei R¹ und R² Wasserstoff sind, R³ Methyl ist, R⁴ OH ist und R⁵ Wasserstoff ist und/oder ein pharmazeutisch verträgliches Salz davon und umfasst das Lösungsmittel Wasser und optional weitere Komponenten (F).

5. Pharmazeutische Zusammensetzung gemäß einem beliebigen der Ansprüche 1 bis 4, wobei die Zusammensetzung das R-Enantiomer der Verbindung der Formel (A) enthält 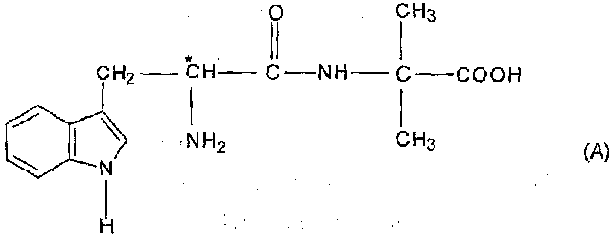 und/oder ein pharmazeutisch verträgliches Salz davon,
und mindestens ein pharmazeutisch verträgliches Cyclodextrin-Derivat (C) und optional einen weiteren wirksamen Bestandteil und/oder weitere Komponenten (F).

6. Pharmazeutische Zusammensetzung gemäß einem beliebigen der Ansprüche 1 bis 5, wobei die Cyclodextrin-Verbindung (C) ausgewählt ist aus der Gruppe bestehend aus:
Alpha-Cyclodextrin, Beta-Cyclodextrin, zufallsalkyliertes Beta-Cyclodextrin, 2-0-Methyl-Beta-Cyclodextrin, Heptakis-(2,6-di-O-methyl)-Beta-Cyclodextrin (Dimethyl-Beta-Cyclodextrin), azetyliertes Dimethyl-Beta-Cyclodextrin, Heptakis-(2,3,6-tri-O-methyl)-Beta-Cyclodextrin (Trimethyl-Beta-Cyclodextrin), 2-Hydroxypropyl-Beta-Cyclodextrin, Sulfo-Alkylether-Beta-Cyclodextrin, Sulfobutylether-Beta-Cyclodextrin, O-Carboxymethyl-O-ethyl-Beta-Cyclodextrin, Glucuronyl-Glucosyl-Beta-Cyclodextrin, Glucosyl-Beta-Cyclodextrin, Maltosyl-Beta-Cyclodextrin, Beta-Cyclodextrinsulphat, Beta-Cyclodextrinphosphat, Gamma-Cyclodextrin, 2-Hydroxypropyl-Gamma-Cyclodextrin, Sulfoalkylether-Beta-Cyclodextrin und Sulfobutylether-Beta-Cyclodextrin.

7. Pharmazeutische Zusammensetzung gemäß einem beliebigen der Ansprüche 1 bis 6, wobei die Verbindung der Formel (I) die R-Konfiguration hat und die Cyclodextrin-Verbindung (C) ausgewählt ist aus Hydroxyalkyl-substituiertem Beta-Cyclodextrin.

8. Pharmazeutische Zusammensetzung gemäß einem beliebigen der Ansprüche 1 bis 7, wobei das molare Verhältnis der Verbindung der Formel (I) und der Cyclodextrin-Verbindung (C) in einem Bereich von 0.1:1 bis 1:20 liegt.

9. Pharmazeutische Zusammensetzung gemäß einem beliebigen der Ansprüche 1 bis 8, wobei die Zusammensetzung eine wässrige flüssige Zusammensetzung, die mindestens 70 Gew.-% Wasser umfasst, ist und wobei die Konzentration der Verbindung der Formel (I) in einem Bereich von 1 mg/ml bis 50 mg/ml liegt.

10. Pharmazeutische Zusammensetzung gemäß einem beliebigen der Ansprüche 1 bis 9, wobei die Zusammensetzung eine wässrige flüssige Zusammensetzung, die als weitere Komponente (F) ein metallhaltiges Konservierungsmittel, insbesondere das Konservierungsmittel Thiomersal umfasst, ist.

11. Pharmazeutische Zusammensetzung gemäß einem beliebigen der Ansprüche 1 bis 10 zur Behandlung einer Störung oder einer Krankheit des Zentralnervensystems.

12. Pharmazeutische Zusammensetzung gemäß einem beliebigen der Ansprüche 1 bis 10 zur Behandlung einer Augenstörung oder -krankheit.

13. Verfahren zur Herstellung einer pharmazeutischen Zusammensetzung umfassend die Schritte des Zusammenmischens mindestens einer Verbindung der Formel (I) 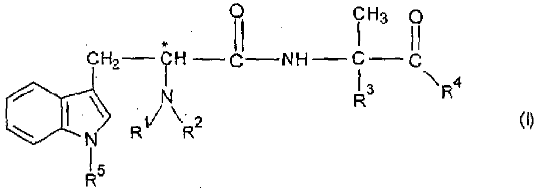 wobei die Reste bedeuten:
R¹ ist Wasserstoff, C₁-C₈-Alkyl, C₃-C₈-Cycloalkyl, C₆-C₁₄-Aryl, Carboxy oder Thiocarb; insbesondere Wasserstoff oder C₁-C₃-Alkyl,
R² ist Wasserstoff C₁-C₈-Alkyl, C₃-C₈-Cycloalkyl, C₆-C₁₄-Aryl-, Carboxy oder Thiocarb; insbesondere Wasserstoff oder C₁-C₃-Alkyl,
R³ ist Wasserstoff oder C₁-C₈-Alkyl, insbesondere Methyl,
R⁴ ist OH oder O-C₁-C₈-Alkyl, insbesondere OH,
R⁵ ist Wasserstoff oder C₁-C₈-Alkyl, insbesondere Wasserstoff;
und/oder ein pharmazeutisch verträgliches Salz davon und/oder eine stereoisomere Form davon,
und mindestens ein pharmazeutisch verträgliches Cyclodextrin-Derivat (C) und optional weitere Komponenten (F).

## Revendications

1. Composition pharmaceutique comprenant au moins un composé de formule (I) 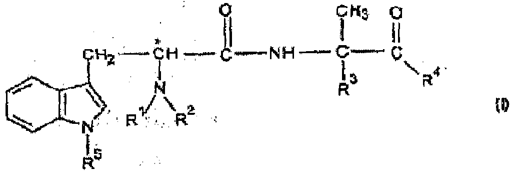 et/ou un sel pharmaceutiquement acceptable en dérivant et/ou une forme stéréoisomère en dérivant,
formule dans laquelle les substituants correspondent à:
- R¹ est un atome d'hydrogène ou un groupe C₁-C₈-alkyle, C₃-C₈-cycloalkyle, C₆-C₁₄-aryle, carboxy ou thiocarb; en particulier un atome d'hydrogène ou C₁-C₃-alkyle,
- R² est un atome d'hydrogène ou un groupe C₁-C₈-alkyle, C₃-C₈-cycloalkyle, C₆-C₁₄-a-ryle, carboxy ou thiocarb; en particulier un atome d'hydrogène ou C₁-C₃-alkyle,
- R³ est un atome d'hydrogène ou un groupe C₁-C₈-alkyle, en particulier un groupe méthyle,
- R⁴ est un groupe OH ou O-C₁-Cs-alkyle, en particulier un groupe OH;
- R⁵ est un atome d'hydrogène ou un groupe C₁-C₈-alkyle, en particulier un atome d'hydrogène;
ladite composition contenant au moins un véhicule pharmaceutiquement acceptable dérivant de cyclodextrine (C) et éventuellement d'autres composants (F).

2. Composition pharmaceutique selon la revendication 1, la composition contenant au moins un composé de formule (I) et/ou un sel pharmaceutiquement acceptable en dérivant, formule dans laquelle les substituants correspondent à:
- R¹ est un atome d'hydrogène ou un groupe C₁-C₈-alkyle, C₃-C₈-cycloalkyle ou C₆-C₁₄-aryle,
- R² est un atome d'hydrogène ou un groupe C₁-C₈-alkyle, C₃-C₈-cycloalkyle ou C₆-C₁₄-aryle,
- R³ est un atome d'hydrogène ou un groupe C₁-C₈-alkyle,
- R⁴ est un groupe OH ou un groupe O-C₁-C₈-alkyle,
- R⁵ est un atome d'hydrogène ou un groupe C₁-C₈-alkyle, en particulier un atome d'hydrogène,
et contenant au moins un autre composant (F).

3. Composition pharmaceutique selon la revendication 1 ou 2, la composition contenant au moins un composé de formule (I) et/ou un sel pharmaceutiquement acceptable en dérivant:
- R¹ est un atome d'hydrogène ou un groupe C₁-C₃-alkyle,
- R² est un atome d'hydrogène ou un groupe C₁-C₃-alkyle,
- R³ est un atome d'hydrogène ou un groupe méthyle,
- R⁴ est OH,
- R⁵ est un atome d'hydrogène ou un groupe C₁-C₈-alkyle, en particulier un atome d'hydrogène,
et contenant de l'eau à titre de solvant et éventuellement d'autres composants (F).

4. Composition pharmaceutique selon l'une quelconque des revendications 1 à 3, la composition contenant un composé de formule (I) et/ou contenant un sel pharmaceutiquement acceptable en dérivant, formule dans laquelle R¹ et R² sont des atomes d'hydrogène, R³ est un groupe méthyle, R⁴ est un groupe OH et R⁵ est un atome d'hydrogène, et contenant de l'eau à titre de solvant et éventuellement d'autres composants (F).

5. Composition pharmaceutique selon l'une quelconque des revendications 1 à 4, dans laquelle- la composition contient l'énantiomère R du composé de formule (A) 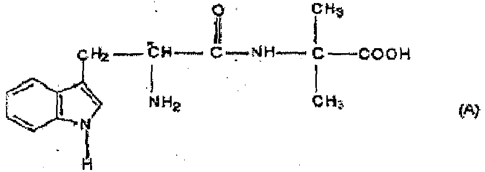 et/ou un sel pharmaceutiquement acceptable en dérivant, et au moins un véhicule pharmaceutiquement acceptable dérivant de cyclodextrine (C) et éventuellement une autre substance active et/ou d'autres composants (F).

6. Composition pharmaceutique selon l'une quelconque des revendications 1 à 5, dans laquelle le composé de cyclodextrine (C) est choisi dans le groupe consistant en: alpha-cyclodextrine, bêta-cyclodextrine, bêta-cyclodextrine alkylée de façon aléatoire, méthyl-bêta-2-O-cyclodextrine, heptakis-(2,6-di-O-méthyl)-bêta-cyclodextrine (diméthyl-bêta-cyclo-dextrine), diméthyl-bêta-cyclodextrine acétylée, heptakis- (2,3,6-tri-O-méthyle)-bêta-cyclodextrine (triméthyl-bêta-cyclodextrine, 2-hydroxypropyl-bêta-cyclo-dextrine, sulfo-alkyléther-bêta-cyclodextrine, sulfobutyléther-bêta-cyclodextrine, O-carboxyméthyl-O-éthyl-bêta-cyclodextrine, glucuronyl-glucosyl-bêta-cyclodextrine, glucosyl-bêta-cyclodextrine, maltosyl-bêta-cyclodextrine, sulfate de bêta-cyclodextrine, le phosphate de bêta-cyclodextrine, gamma-cyclodextrine, 2-hydroxypropyl-gamma-cyclodextrine, sulfoalkylether-bêta-cyclodextrine et sulfobutyléther-bêta-cyclodextrine.

7. Composition pharmaceutique selon l'une quelconque des revendications 1 à 6, dans laquelle- le composé de formule (I) a la configuration R et le composé de cyclodextrine (C) est choisi parmi les béta-cyclodextrines à substituants hydroxyalkyles.

8. Composition pharmaceutique selon l'une quelconque des revendications 1 à 7, dans laquelle- le rapport molaire du composé de formule (I) au composé de cyclodextrine (C) est dans l'intervalle de 0,1/1 à 1/20.

9. Composition pharmaceutique selon l'une quelconque des revendications 1 à 8, dans laquelle- la composition est une composition liquide aqueuse comprenant au moins 70% en poids d'eau et dans laquelle- la concentration du composé de formule (I) se situe dans la plage allant de 1 mg/ml à 50 mg/ml.

10. Composition pharmaceutique selon l'une quelconque des revendications 1 à 9, dans laquelle- la composition est une composition liquide aqueuse comprenant comme autre composant (F) un agent de conservation contenant du métal, en particulier comme agent de conservation le thiomersal.

11. Composition pharmaceutique selon l'une quelconque des revendications 1 à 10 pour le traitement d'un trouble ou d'une maladie du système nerveux central.

12. Composition pharmaceutique selon l'une quelconque des revendications 1 à 10 pour le traitement d'un trouble ophtalmique ou d'une maladie ophtalmique.

13. Procédé de préparation d'une composition pharmaceutique comprenant les étapes consistant à mélanger ensemble au moins un composé de formule (I) et/ou un sel pharmaceutiquement acceptable en dérivant, et/ou une forme stéréoisomère n dérivant, 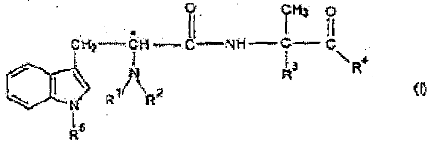 formule dans laquelle les substituants correspondent à::
- R¹ est un atome d'hydrogène ou un groupe C₁-C₈-alkyle, C₃-C₈-cycloalkyle, C₆-C₁₄-aryle, carboxy ou thiocarb; en particulier un atome d'hydrogène ou C₁-C₃-alkyle,
- R² est un atome d'hydrogène ou un groupe C₁-C₈-alkyle, C₃-C₈-cycloalkyle, C₆-C₁₄-aryle, carboxy ou thiocarb; en particulier un atome d'hydrogène ou C₁-C₃-alkyle,
- R³ est un atome d'hydrogène ou un groupe C₁-C₈-alkyle, en particulier un groupe méthyle,
- R⁴ est un groupe OH ou un groupe O-C₁-C₈-alkyle, en particulier OH,
- R⁵ est un atome d'hydrogène ou un groupe C₁-C₈-alkyle, en particulier un atome d'hydrogène;
et au moins un véhicule pharmaceutiquement acceptable dérivé de cyclodextrine (C) et éventuellement d'autres composants (F).
